Europäisches Patentamt

European Patent Office

Office européen des brevets

(11) Veröffentlichungsnummer: **0 340 307**
A1

## (12) EUROPÄISCHE PATENTANMELDUNG

Veröffentlicht nach Art. 158 Abs. 3 EPÜ

(21) Anmeldenummer: 88907444.9

(22) Anmeldetag: 27.04.88

Daten der zugrundeliegenden internationalen Anmeldung:

(86) Internationale Anmeldenummer:
PCT/SU88/00104

(87) Internationale Veröffentlichungsnummer:
WO89/03859 (05.05.89 89/10)

(51) Int. Cl.³: **C 08 L 39/06**
**C 08 K 7/02, A 61 K 9/70**
**A 61 L 15/03, A 61 M 37/00**

(30) Priorität: 23.10.87 SU 4316266

(43) Veröffentlichungstag der Anmeldung:
08.11.89 Patentblatt 89/45

(84) Benannte Vertragsstaaten:
CH DE FR GB IT LI NL SE

(71) Anmelder: VSESOJUZNY
NAUCHNO-ISSLEDOVATELSKY INSTITUT
BIOTEKHNOLOGII OBIEDINENIA 'BIOTEKHNOLOGIA'
pr. Nauchny, 8
Moscow, 117246(SU)

(71) Anmelder: VSESJUZNY KARDIOLOGICHESKY
NAUCHNY TSENTR AKADEMII MEDITSINSKIKH NAUK
SSSR
ul. 3 Cherepkovskaya, 15a
Moscow, 121552(SU)

(71) Anmelder: PROIZVODSTVENNO-EXPERIMENTALNY
ZAVOD "SANITAS" NAUCHNO-PROIZVODSTVENNOGO
OBIEDINENIA "FERMENT"
ul. Lenina, 3
Kaunas, 233000(SU)

(72) Erfinder: VASILIEV, Alexandr Evgenievich
ul. Zamorenova, 11a-17
Mpscow, 123376(SU)

(72) Erfinder: PLATE, Nikolai Alfredovich
Leninsky pr., 61-16
Moscow, 117333(SU)

(72) Erfinder: FELDSHTEIN, Mikhail Maiorovich
pr. Leninsky, 102-45
Moscow, 117415(SU)

(72) Erfinder: SHVARTS, Iosif Shimonovich
ul. Marshala Ustinova, 3-184
Moscow, 121609(SU)

(72) Erfinder: TITOV, Alexandr Petrovich
ul. Teply Stan, 21-4-66
Moscow, 117133(SU)

(72) Erfinder: MAXIMENKO, Olga Olegovna
ul. Osennaya, 4-1-303
Moscow, 121609(SU)

(54) ZUSAMMENSETZUNG EINER POLYMER-DIFFUSIONSMATRIZE ZUR TRANSDERMALEN VERABREICHUNG VON ARZNEIMITTELN.

(57) Zusammensetzung einer polymeren Diffusionsmatrix für transdermale Einführung von Arzneistoffen enthält 100 Masseanteile Polyvinylpyrrolidon mit einer Molekularmasse von 500 Tsd bis 1500 Tsd. von 33 bis 100 Masseanteilen Polyäthylenglykol mit einer Molekularmasse von 300 bis 600 und von 0,6 bis 66 Masseanteilen eines Arzneistoffes.

./...

(72) Erfinder: TOKHMAKHCHI, Viktoria Nikolaevna
pr. Kutuzovsky, 1/7-97
Moscow, 121248(SU)

(72) Erfinder: MALKHAZOV, Lev Borisovich
ul. Krasnoarmeiskaya, 23-80
Moscow, 125319(SU)

(72) Erfinder: OGANOV, Rafael Gegamovich
ul. B.Gruzinskaya, 39-147
Moscow, 123056(SU)

(72) Erfinder: METELITSA, Vladimir Isaakovich
Jubileiny pr., 35-186 Moskovskaya obl.
Khimki, 141400(SU)

(72) Erfinder: PIOTROVSKY, Vladimir Konstantinovich
ul. Akademika Skryabina, 3-1-59
Moscow, 109377(SU)

(72) Erfinder: DUDENAS, Gendrik Eduardovich
ul. Salomei Neris, 32-4
Kaunas, 233018(SU)

(72) Erfinder: MAKAUSKAS, Ionas Ionovich
ul. G.Borisos, 16-16
Kaunas, 233005(SU)

(72) Erfinder: BERTULIS, Albert Pyatrovich
ul. Angarietisa, 15-1
Kaunas, 233018(SU)

(74) Vertreter: von Füner, Alexander, Dr.
Patentanwälte v. Füner, Ebbinghaus, Finck Mariahilfplatz
2 & 3
D-8000 München 90(DE)

# ZUSAMMENSETZUNG EINER POLYMEREN DIFFUSIONSMATRIX ZUR TRANSDERMALEN EINFÜHRUNG VON ARZNEISTOFFEN

## Gebiet der Technik

Die vorliegende Erfindung bezieht sich auf polymere Arzneiformen mit kontrollierbarer Zuführung von Arzneistoffen, und insbesondere betrifft sie eine Zusammensetzung einer polymeren Diffusionsmatrix für transdermale Einführung von Arzneistoffen.

## Zugrundeliegender Stand der Technik

In der letzten Zeit werden umfassend transdermale therapeutische Systeme entwickelt und von der Industrie hergestellt, die eine Arzneiform der neuen Generation für eine prolongierte und kontinuierliche Zuführung eines Arzneistoffes unmittelbar durch die nicht beschädigte Haut nach einem vorgebenen Programm dem Organismus eines Patienten darstellen.

Die Anstrengungen der Forscher, die sich mit Entwicklung von transdermalen therapeutischen Systemen befassen, sind auf die Einbeziehung eines breiten Kreises von Arzneistoffen in diese Systeme konzentriert, wofür es notwendig ist, polymere Diffusionsmatrizen zu entwickeln, die sich durch eine hohe Geschwindigkeit der Freilegung von Arzneistoffen auszeichnen. Die von uns durchgeführten Forschungen der Kinetik der Permeabilität von Hydralazin in vitro durch die Leichenhaut eines Menschen an der Trenngrenze der Medien unterschiedlicher Polarität zeigten, daß die Geschwindigkeit der transdermalen Zuführung eines Arzneistoffes aus den Medien, die die polymeren Matrizen modellieren, durch den Verteilungskoeffizient des Arzneimittels zwischen der jeweiligen Matrix und einer Rezeptorlösung, die das Blutplasma eines Menschen modeliert, sowie durch den Grad der Löslichkeit des Arzneistoffes in der Matrix geregelt wird. Dabei ist gerade die Löslichkeit eines Arzneistoffes in der Matrix für viele Arzneimittel von entscheidender Bedeutung durch die Erhöhung der Löslichkeit des Arzneistoffes in der Matrix gelingt es in der Regel, die Geschwindigkeit der transdermalen Zuführung des Arzneistoffes zu steigern, was in voller Über-

einstimmung mit dem Fickschen Gesetz und den theoretischen Voraussagen steht. Angesichts dessen aber, daß eine bedeutende Menge von Arzeinstoffen ionogene organische Stoffe darstellen, die eine höhere Löslichkeit in hydrophilen Medien als in lypophilen aufweisen, sollen polymere Diffusionsmatrizen auf der Grundlage von hydrophilen Polymeren universaler als die Matrizen auf der Grundlage von hydrophoben Polymeren sein. In Wirklichkeit erlauben es die hydrophilen polymeren Matrizen, eine hohe Geschwindigkeit der transdermalen Zuführung von Arzneisstoffen von ionogener, hydrophiler und diphiler Herkunft zu erreichen, dagegen erlauben es die Matrizen auf der Grundlage von hydrophoben Polymeren, beispielsweise von Polyisobutylenkautschuk, in der Regel nicht, Geschwindigkeitswerte der transdermalen Zuführung von Arzneistoffen oberhalb von 5 bis 10 g/cm$^2$·h zu erreichen.

Bekannt ist eine universale hydrophile polymere Diffusionsmatrix, die für transdermale Einführung eines breiten Kreises von Arzneistoffen mit einer Geschwindigkeit geeignet ist, die die Zufuhrgeschwindigkeit von Arzneistoffen aus hydrophoben Matrizen übersteigt, sie enthält von 2 bis 15 Masse% Polyvinylalkohol mit einer Molekularmasse von 100 Tsd bis 150 Tsd, von 2 bis 60 Masse% Glyzerin, von 2 bis 10 Masse% eines wasserlöslichen Polymeres mit hydratierbaren Gruppen, beispielsweise Polyvinylpyrrolidon, mit einer Molekularmasse von 20 Tsd. bis 60 Tsd, einen Arzneistoff und Wasser (von 36 bis 73 Masse% ) (US, A, 4466953).

Zu den Nachteilen dieser Zusammensetzung der Matrix gehören:

- unzureichend hohe Zufuhrgeschwindigkeit von Arzneistoffen;

- Verringerung der Zufuhrgeschwindigkeit von Arzneistoffen aus der jeweiligen Matrix proportional dem Quadratwurzel der Zeit (was durch die Gleichung von T.Higuchi beschrieben wird, während es mehr die Freilegung des Arzneistoffes mit einer Geschwindigkeit vorzuziehen wäre, die von der Zeit nicht abhängig ist);

0340307

- ungenügende Wirkungsdauer der Matrix (etwa 24 Stunden), was die Folge der Abhängigkeit der Zuführgeschwindigkeit eines Arzneistoffes von dem Quadratwurzel der Zeit (sustained release), sowie die Unmöglichkeit der Einführung hoher Konzentrationen an Arzneistoffen in die Zusammensetzung einer Matrix;

- niedriger Ausnutzungskoeffizient des Arzneistoffes, weil lediglich 10% des in die Zusammensetzung der Matrix eingeführten Arzneimittels in den Organismus eines Patienten eintritt;

- Fehlen von Adhäsionseigenschaften bei einer Matrix, weshalb die Unveränderlichkeit der Kontaktfläche einer Diffusionsmatrix mit der Haut nicht gesichert wird, und als Folge eine starke sprunghafte Änderung der Konzentration des Arzneimittels im Blut eines Patienten entsteht. Die Befestigung der Matrix an der Haut verlangt außerdem Fixierungsvorrichtungen, solcher wie Binden, Armbänder, Bandagen und anderes mehr.

Offenbarung der Erfindung

Der vorliegenden Erfindung liegt die Aufgabe zugrunde, durch die Wahl von Polymeren, die in einem bestimmten Verhältnis genommen werden, eine solche Zusammensetzung einer polymeren Diffusionsmatrix für transdermale Einführung von Arzneistoffen zu entwickeln, die eine hohe und konstante Zufuhrgeschwindigkeit verschiedener Arzneimittel dem jeweiligen Organismus, einen hohen Ausnutzungskoeffizienten derselben bei einer langen Wirkungsdauer gewährleistete und eine hohe Adhäsion mit der Haut aufwies.

Die genannte Aufgabe wird dadurch gelöst, daß in der Zusammensetzung der polymeren Diffusionsmatrix für transdermale Einführung von Arzneistoffen, die ein Bindemittel, Polyvinylpyrrolidon, ein Plastifizierungsmittel und einen Arzneistoff enthält, erfindungsgemäß, als Bindemittel Polyvinylpyrrolidon mit einer Molekularmasse von 500 Tsd. bis 1.500 Tsd, als Plastifizierungsmittel, Polyäthylenglykol mit einer Molekularmasse von 300 bis 600 bei folgendem Verhältnis der genannten Komponenten in Masse-

anteilen vorhanden sind:

| | |
|---|---|
| Polyvinylpyrrolidon | 100 |
| Polyäthylenglykol | - von 33 bis 100 |
| Arzneistoff | von 0,6 bis 66. |

Durch die beanspruchte Zusammensetzung ist es möglich, eine Aghäsionsmatrix zu entwickeln, die es ermöglicht, Arzneistoffe mit einer ausreichend hohen und konstanten sowie nicht zeitabhängigen Geschwindigkeit transdermal einzuführen.

Der Ausnutzungskoeffizient eines Arzneistoffes vergrößert sich bei der Anwendung der beanspruchten Erfindung auf das 8fache im Vergleich mit der bekannten Matrix (US, A, 4466953). Die Wirkungsdauer der Matrix, die in der angemeldeten Erfindung vorgeschlagen wird, übertrifft auf das 7fache die Wirkung der bekannten Matrix (US, A, 4466953).

Bei der Einführung von Propranolol aus einer Matrix, die die beanspruchte Zusammensetzung aufweist, beträgt die Geschwindigkeit seines Eintritts (durch die Leichenhaut) 47 $\mu$g/cm$^2$.h, das Nitroglyzerin tritt (durch die Leichenhaut) mit einer Geschwindigkeit von 39 $\mu$g/cm$^2$.h ein.

Für die Aufrechterhaltung der erreichten Eigenschaften einer Matrix unter erhöhter Schweißabsonderung sowie für die weitere Steigerung der Zufuhrgeschwindigkeit von Arzneistoffen aus der Matrix ist es zweckmäßig, erfindungsgemäß, daß die Zusammensetzung zusätzlich einen hydrophilen unlöslichen faserigen Füllstoff auf der Grundlage von Zellulose in einer Menge von 5 bis 200 Masseanteilen enthält. Zweckmäßigerweise, soll die vorgeschlagene Zusammensetzung, erfindungsgemäß, zusätzlich Promotoren der Permeabilität der Arzneimittel durch die Haut in einer Menge von 1 bis 10 Masseanteilen enthalten. Erfindungsgemäß, ist es zweckmäßig, daß die vorgeschlagene Zusammensetzung zusätzlich Lösungsmittel für Arzneistoffe in einer Menge von 5 bis 15 Masseanteilen enthält.

Weitere Ziele und Vorteile der vorliegenden Erfindung werden aus der nachstehenden ausführlichen Beschreibung

0340307

der Zusammensetzung der polymeren Diffusionsmatrix für transdermale Einführung von Arzneistoffen und aus Beispielen für die Ausführung dieser Zusammensetzung ersichtlich.

Beste Ausführungsvariante der Erfindung

In Übereinstimmung mit der angemeldeten Erfindung weist die Zusammensetzung einer polymeren Diffusionsmatrix für transdermale Einführung von Arzneistoffen in Masseanteilen auf:

| | |
|---|---|
| Polyvinylpyrrolidon mit einer Molekularmasse von 500 Tsd. bis 1.500 Tsd. | 100 |
| Polyäthylenglykol mit einer Molekularmasse von 300 bis 600 | von 33 bis 100 |
| Arzneistoff | von 0,6 bis 66 |

und notwendigenfalls
einen hydrophilen unlöslichen
faserigen Füllstoff auf der
Grundlage von Zellulose            von 5
bis 200.

Das in der vorliegenden Erfindung als Bindemittel zum Einsatz kommende Polyvinylpyrrolidon mit einer Molekularmasse von 500 Tsd. bis 1.500 Tsd. stellt ein hygroskopisches gelbliches Pulver mit schwachem Geruch, ohne Geschmack dar; es ist in Wasser, Alkohol, in aromatischen Kohlenwasserstoffen und in Chloroform löslich; es ist in Äther und in aliphatischen Kohlenwasserstoffen unlöslich.

Die Verwendung von Polyvinylpyrrolidon mit einer Molekularmasse unter 500 Tsd. in der der Matrix gewährleistet nicht die erforderliche Zufuhrgeschwindigkeit von Arzneistoffen und ermöglicht es nicht, eine Matrix mit erforderlichen mechanischen Eigenschaften und Adhäsionsfähigkeit herzustellen, und die Verwendung des Polyvinylpyrrolidons mit einer Molekularmasse oberhalb von 1.500 Tsd. verursacht die Entstehung von Matrizen mit unzureichender Elastizität.

Als Plastifizierungsmittel und Regelungsmittel der Adhäsion wird Polyäthylenglykol mit einer Molekularmasse von 300 bis 600 verwendet, das eine farblose zähflüssige Flüssigkeit mit einem schwachen spezifischen Geruch darstellt. Es ist in Alkohl, Wasser und in vielen organischen Lösungsmitteln gut löslich.

Die Verwendung des Polyäthylenglykols mit einer Molekularmasse oberhalb von 600 verringert die Elastizität und die Adhäsionsfähigkeit der jeweiligen Matrix. Die Verwendung des Polyäthylenglykols mit einer Molekularmasse unter 300 ermöglicht es nicht, eine Matrix mit erforderlicher Festigkeit herzustellen.

Eine hohe konstante Geschwindigkeit der Zuführung eines Arzneistoffes durch die Haut wird durch das gleichzeitige Vorliegen des genannten Polyvinylpyrrolidons und des genannten Polyäthylenglykols in der Zusammensetzung einer Matrix, die in den obengenannten Mengen genommen werden, was einem Verhältnis des Polyäthylenglykols zum Polyvinylpyrrolidon gleich 1 : 1 bis 1 : 3 entspricht.

Die Veränderung des Verhältnisses des Polyäthylenglykols zum Polyvinylpyrrolidon in der Zusammensetzung der Matrix in Richtung der Verringerung des Gehalts an Polyvinylpyrrolidon führt zur Entstehung von flüssigen Matrizen und in Richtung der Vergrößerung des Gehalts an Polyvinylpyrrolidon (oberhalb der angemeldeten Menge) ruft die Senkung der Geschwindigkeit des Austritts des Arzneistoffes, die Verringerung von Adhäsionskennlinien der Matrix sowie die Verschlechterung ihrer mechanischen Eigenschaften hervor.

Durch die beanspruchte Erfindung ist es möglich, als Arzneistoff Verbindungen mit unterschiedlichem chemischem Aufbau zu verwenden: Derivate der aliphatischen Reihe (beispielsweise Nitroglyzerin), der aromatischen Reihe (beispielsweise Anaprilin), der azyklischen Reihe (Dinitratisosorbid/ und der heterozyklischen Reihe (Nifedipin).

Die Menge des Arzneistoffes in der Zusammensetzung einer Matrix wird durch seine Löslichkeit in einem Diffusionsmedium und durch die erforderliche Wirkungsdauer der Matrix ermittelt.

In Übereinstimmung mit dieser Ausführungsvariante der vorliegenden Erfindung unter Bedingungen einer erhöhten Schweißabsonderung bei einer langen Anwendung der Matrix (über 24 Stunden) ist es zweckmäßig, zusätzlich einen Füllstoff in die Zusammensetzung der Matrix einzuführen.

In Übereinstimmung mit dieser Ausführungsvariante der vorliegenden Erfindung kann die Geschwindigkeit der transdermalen Einführung von Arzneistoffen durch die Einführung eines hydrophilen unlöslichen faserigen Füllstoffes auf der Grundlage von Zellulose in die Matrix erhöht werden, dessen Einführung auch ermöglicht, die Gebrauchseigenschaften und die Stabilität der Eigenschaften der Matrix unter Bedingungen der Schweißabsonderung und eines langen Tragens wesentlich zu verbessern.

Die Steigerung der Geschwindigkeit der transdermalen Zuführung von Arzneistoffen ermöglicht es, die Fläche einer Matrix zu verringern, die für die Erreichung des erforderlichen Konzentrationsstandes an einem Arzneistoff im Blutplasma eines Patienten notwendig ist, oder bei einer gleichen, im Vergleich zum Prototyp, Fläche der Matrix eine höhere Konzentration an Arzneistoff im Blutplasma zu schaffen. Hierdurch wird es bei der Verwendung einer Matrix mit dem erfindungsgemäßen Füllstoff möglich, die Arzneistoffe zu verwenden, für die bei der Verwendung der bekannten Matrizen der therapeutische Konzentrationsstand im Blutplasma nicht erreicht werden kann.

Als ein hydrophiler unlöslicher faseriger Füllstoff auf der Grundlage von Zellulose und ihren Derivaten werden gewebte und ungewebte Papier- und Baumwollstoffe, solche beispielsweise wie Filterpapier, Papierabsorptionsmittel, Sonderpapier und Stapelfaserstoff, verwendet. Die Kenndaten der Füllstoffe sind in der Tabelle 1 angeführt.

Tabelle 1

| Füllstoff | Stärke, $\mu m$ | Masse, 1 cm$^2$, mg | Wasserauf- nahme, mg H$_2$O/cm$^2$ | Verhält- nis der Wasser- aufnahme zur Masse |
|---|---|---|---|---|
| 1 | 2 | 3 | 4 | 5 |
| Gewebter Baumwollstoff | 340 | 13,0 | 33,35 | 2,57 |
| Gewebter Baumwollstoff | 220 | 9,25 | 21,55 | 2,33 |
| Gewebter Baumwollstoff | 220 | 6,25 | 43,6 | 6,98 |
| Gewebter Baumwollstoff | 250 | 16,8 | 36,6 | 2,18 |
| Gewebter Baumwollstoff | 290 | 14,65 | 28,9 | 1,97 |
| Papier | 100 | 3,4 | 5,4 | 1,59 |
| Papier | 290 | 3,3 | 20 | 6,06 |
| Filterpapier | 300 | 9,8 | 11 | 1,12 |

Bessere Eigenschaften weisen die gewebten Füllstoffe mit einer maximalen Wasseraufnahme je Einheit der Masse auf.

Die in der vorliegenden Erfindung vorgeschlagene Zusammensetzung kann Promotoren der Permeabilität von Arzneistoffen durch die Haut, beispielsweise, 1-Dodezyl-(azazykloheptan-2-on), N,N-Dimethyl-m-toluamid, Dimethylsulfoxid, N-Methylpyrrolidon enthalten. Die genannten Komponenten sind in der erfindungsgemäßen Zusammensetzung in einer Menge von 1 bis 10 Masseanteilen enthalten.

Zur Verbesserung der Löslichkeit der Arzneistoffe in dem Diffusionsmedium der Matrix ist die Einbeziehung solcher Stoffe wie Twine und Tritone in die Zusammensetzung der Matrix möglich. Der Gehalt an diesen Stoffen in der erfindungsgemäßen Zusammensetzung ist üblicherweise von 5 bis 15 Masseanteile gleich.

Aus der in der beanspruchten Erfindung angemeldeten Zusammensetzung erhält man eine polymere Diffusionsmatrix für die transdermale Einführung der Arzneistoffe durch Auftragen einer Schicht dieser Zusammensetzung auf eine Unterlage. Als Unterlage wird üblicherweise eine Folie, beispielsweise aus Polyäthylenterephthalat, Polyäthylen und Polyvinylchlorid, verwendet. Vorzugsweise soll eine Polyäthylenterephthalfolie verwendet werden, die mit einer Schicht aus Metallaluminium von der der Matrix gegenüberliegenden Seite überzogen ist.

Das Auftragen einer Schicht der beanspruchten Zusammensetzung erfolgt üblicherweise im Gießverfahren aus einer Lösung. Als Lösungsmittel wird hierfür Äthylalkohol, Wasser oder ihr Gemisch verwendet.

Eine getrocknete Schicht mit einer Stärke von 50 $\mu$m bis 1 mm der genannten Zusammensetzung stellt gerade eine Matrix dar. Das ist eine elastische durchsichtige Polymerfolie, die eine hohe Adhäsion gegenüber Haut und Polymerstoffen, die als Unterlage eingesetzt werden, aufweist. Die Adhäsion der hergestellten Matrix beträgt von 51 bis 81 n/m, was für eine zuverlässige Befestigung einer Matrix an der Haut eines Patienten ausreichend ist, das heißt für die Unterhaltung der Zufuhrgeschwindigkeit eines Arzneistoffes auf einem beständigen Niveau ohne zusätzliche Mittel zum Fixieren der Matrix.

Zum Schutz der adhäsionsschicht der hergestellten Matrix vor Beschädigung und Verschmutzung bei der Lagerung wird ihre Oberfläche mit einem Antiadhäsionsschutzstoff, beispielsweise mit silikonisiertem Antiadhäsionspapier laminiert, das unmittelbar vor dem Auftragen einer Matrix auf die Haut abgezogen wird.

Die aus der erfindungsgemäßen Zusammensetzung hergestellte Matrix bewirkt den Ausnutzungskoeffizienten des Arzneistoffes (die Menge des aus der Matrix in den jeweiligen Organismus eingetretenen Arzneistoffes), der 82% erreicht. Dabei erfolgt die Zuführung des Arzneistoffes dem Organismus mit einer konstanter, von der Zeit nicht abhängigen Geschwindigkeit. So ist die Geschwindigkeit der

- 10 -

transdermalen Zuführung von Propranolol aus einer polymeren Diffusionsmatrix mit der erfindungsgemäßen Zusammensetzung gleich 47 $\mu$g/cm$^2$.h (und aus einer Matrix, die einen Füllstoff enthält - 55 $\mu$g/cm$^2$.h).

Die Geschwindigkeit der transdermalen Zuführung von Nitroglyzerin aus einer polymeren Diffusionsmatrix der angemeldeten Zusammensetzung ist gleich 39 $\mu$g/cm$^2$.h. Die Wirkungsdauer der polymeren Matrix der in der Erfindung angemeldeten Zusammensetzung erreicht 7 Tage. Die erfindungsgemäße Zusammensetzung der polymeren Diffusionsmatrix läßt sich durch Flüssigkeits-Chromatografie kontrollieren.

Pharmakologische Eigenschaften der Matrizen, die aus der angemeldeten Zusammensetzung hergestellt werden, wurden "in vitro" und "in vivo" geprüft.

Die Prüfungen der Matrizen "in vivo" erfolgten an rassenlosen weißen Ratten, an Kaninchen und Minischweinchen.

Bei der Prüfung der Matrix, die auf der Grundlage einer Zusammensetzung hergestellt wurde, die 100 g Polyvinylpyrrolidon mit einer Molekularmasse von 500 Tsd. bis 1500 Tsd.,54,5 g Polyäthylenglykol mit einer Molekularmasse von 400, 12 g Hydralazin enthält, und die eine Fläche von 3,14 cm$^2$ hat, wurde diese den rassenlosen weiß--en Ratten auf die abgeschorene Körperoberfläche im Rückenbereich aufgeklebt. Der Kontrollgruppe von Tieren führte man eine Apressin-Injektion in einer Dosis von 0,5 mg/kg ein. In den beiden Ratten-Gruppen ermittelte man den systolischen arteriellen Druck an der Schwanzader mit Hilfe eines manometrischen Gebers. Die Injektion verringert den systolischen arteriellen Druck um 25 bis 30% innerhalb von 15 Minuten, und in 60 Minuten kehrt der Druck praktisch zum Ausgangsniveau zurück. Nach dem Aufkleben einer Matrix mit Hydralazin verringert sich der systolische arterielle Druck während der ersten Stunde um 10 bis 20%, der maximale Wirkungseffekt wird in 2 bis 4 Stunden (24%) erreicht, zum Ausgangsniveau kehrt der Druck am fünften Tag (Tabelle 2) zurück.

- 11 -

Zum Nachweis des Hydralazins im Blut wurden Experimente an Minischweinchen durchgeführt. Die Haut eines Laborschweines wurde in der Bauchgegend mit einem Wattebausch, der mit Wasser angefeuchtet wurde, abgerieben und man legte an diese Gegend eine Diffusionsmatrix an. Dabei wurde festgestellt, daß die auf der Grundlage der obengenannten Zusammensetzung hergestellte Matrix unverzüglich kleben bleibt, während eine Matrix, die auf der Grundlage der bekannten Zusammensetzung (US, A, 4466953) hergestellte wurde, an der Haut des Tieres mit einer Mullbinde fixiert werden sollte. Der Gehalt an Hydralazin im Blut des Tieres ermittelte man mittels Gas-Chromatografie-Analyse. Die auf der Grundlage der bekannten Zusammensetzung (US, A, 4466953) hergestellte Matrix wurde an ein Schwein mit einem Gewicht von 23 kg aufgeklebt; die Matrixfläche betrug 50 cm$^2$ (2,2 cm$^2$/kg). Die auf der Grundlage der obengenannten Zusammensetzung hergestellte Matrix wurde auf ein Schwein mit einem Gewicht von 34 kg aufgeklebt; die Matrixfläche beträgt 77 cm$^2$ (2,2 cm$^2$/kg). Die Prüfergebnisse sind in der Tabelle 3 angeführt. Hervorzuheben ist, daß der Gehalt an Hydralazin im Blut eines Tieres beim Experiment mit einer Diffusionsmatrix, die auf der Grundlage der bekannten Zusammensatzung (US, A, 4466953) hergestellt wurde, bedeutend schwankt, weil infolge des Fehlens von Adhäsionseigenschaften in dieser Matrix ihre Berührungsfläche mit der Haut mit der Zeit durch die Bewegungen von Tieren sich verändert.

Aus den in der Tabelle 3 angeführten Angaben ist zu ersehen, daß der Gehalt des Blutes an Hydralazin innerhalb der ersten Stunde nach dem Auftragen der Matrix auf die Haut erhöht ist (Maximaldosis), wonach er sich verringert und den unverändert bleibenden Stand erreicht, der im Verlaufe der nächstfolgenden mehreren Tagen konstant bleibt.

Tabelle 2

Untersuchung des Einflusses einer polymeren Diffusionsmatrix mit Hydralazin auf den systolischen arteriellen Druck

| lfd. Nr. der Tiere | Herzton-kennziffer, Torr | Intervalle der Veränderung des systolischen arteriellen Druckes, min | | | | |
|---|---|---|---|---|---|---|
| | | 15 | 30 | 45 | 60 | 120 |
| 1 | 2 | 3 | 4 | 5 | 6 | 7 |
| 1 | 110 | | | | 100 | 90 |
| 2 | 100 | | | | 100 | |
| 3 | 115 | | | | 110 | 80 |
| 4 | 110 | | | | 80 | 80 |
| 5 | 100 | | | | 90 | 90 |
| 6 | 105 | | | | 90 | 90 |
| 7 | 100 | | | | 90 | 90 |

Durchschnitverschiebungen des systolsichen arteriellen Druckes in % zum Ausgangsniveau: 10,7  18,0

Fortsetzung der Tabelle 2

| | 2 | 3 | 4 | 5 | 6 | 7 |
|---|---|---|---|---|---|---|
| Injektion | | | | | | |
| 8 | 100 | 80 | 85 | 90 | 105 | 100 |
| 9 | 110 | 80 | 90 | 80 | 80 | 110 |
| 10 | 110 | 80 | 75 | 95 | 95 | 115 |
| 11 | 110 | 75 | 70 | 90 | 70 | 110 |
| 12 | 110 | 75 | 75 | 90 | 105 | 105 |

Durchschnittsverschiebungen des systolischen arteriellen Druckes in % zum Ausgangs druck: 27,8  26,9  17,6   15,7   0

Fortsetzung der Tabelle 2

| lfd. Nr. des Tieres | Intervalle der Messungen des systolischen arteriellen Druckes, min | | | | | |
|---|---|---|---|---|---|---|
| | 180 | 240 | 300 | 360 | 720 | 1440 |
| 1 | 8 | 9 | 10 | 11 | 12 | 13 |
| 1 | 80 | 85 | | 90 | 90 | 85 |
| 2 | 80 | 80 | | | 80 | 85 |
| 3 | 80 | 80 | | 80 | 80 | 85 |
| 4 | 80 | 80 | | 75 | 80 | 75 |
| 5 | | 85 | 80 | 80 | 75 | 80 |
| 6 | 90 | 90 | | 80 | 80 | 75 |
| Durchschnittliche Verschiebungen des systolischen arteriellen Druckes in % zum Ausgangsdruck | 21,2 | 20,3 | | 23,6 | 22,9 | 23,6 |

Tabelle 3

Gehalt an Hydralazin im Blut von Minischweinchen bei transdermaler Zuführung desselben aus einer Diffusionsmatrix

| lfd. Nr. | Analysierbare Kenngröße | Dauer der Probeentnahme, h | | | | |
|---|---|---|---|---|---|---|
| | | 0,5 | 0,6 | 1 | 2 | 4 |
| 1 | 2 | 3 | 4 | 5 | 6 | 7 |
| | Die auf der Grundlage der erfindungsgemäßen Zusammensetzung hergestellte Matrix | | | | | |
| 1 | Gehalt des Blutes, an Hydralazin $\mu$m/ml | 20 | 10 | 3,5 | 3,9 | 3,8 |
| | Die auf der Grundlage der bekannten Zusammensetzung hergestellte Matrix (US, A, 4466953) | | | | | |
| 2 | Gehalt des Blutes an Hydralazin, $\mu$m/ml | 1,3 | | 1,2 | 0,7 | Spuren |

Fortsetzung der Tabelle 3

| lfd. Nr. | Dauer der Probeentnahme, h | | | | Durchschnitt- liche unver- ändert blei- bende Ge- schwindigkeit | Abweichung von dem un- verändert bleibenden Wert, % |
|---|---|---|---|---|---|---|
| | 6 | 12 | 24 | 48 | | |
| 1 | 8 | 9 | 10 | 11 | 12 | 13 |
| 1 | | 4,0 | 3,9 | 4,1 | 73,8±2,1 | 2,9 |
| 2 | 0,9 | 0,5 | 0,4 | Spuren | 7,3±6,4 | 87,7 |

Die auf der Grundlage einer Zusammensetzung hergestellte Matrix, die 100 g Polyvinylpyrrolidon mit einer Molekularmasse von 500 Tsd. bis 1500 Tsd. 52,2 g Polyäthylenglykol mit einer Molekularmasse von 400 und 27,2 g Propranolol enthält, wurde in einem Experiment an sechs Kaninchen untersucht (Tabelle 4).

Die Konzentration an Propranolol im Blutplasma von
Kaninchen erhöht sich innerhalb von 24 Stunden kontinuierlich und erreicht den unverändert bleibenden Wert, im
weiteren bleibt sie konstant während sechs Tage seit dem
Zeitpunkt der Applikation der Matrix an der Haut. Ähnliche
Angaben wurden in Versuchen an Ratten und Katzen erzielt.
Die erzielten Ergebnisse der experimentellen Untersuchung
der Pharmakokinetik einer Matrix mit Propranolol entsprechen voll und ganz den theoretischen Berechnungen, in denen es nachgewiesen wurde, daß die unverändert bleibende
Konzentration an demselben im Blut bei der transdermalen
Einführung nach 24 Stunden seit dem Zeitpunkt des Beginns
der Zuführung des Propranolols durch die Haut erreicht
werden kann. Die Pharmakodynamik der transdermalen Matrizen
mit Propranolol untersuchte man an den gleichen drei Arten
von Tieren. Propranolol ruft bei seiner transdermalen Zuführung aus Diffusionsmatrizen eine eindeutige Senkung der
Herzkontraktion sowie die Senkung des arteriellen Druckes
hervor. Die spezifische $\beta$ -adrenoblockierende Aktivität
der Diffusionsmatrizen mit Propranolol wurden in Experimenten mit einem $\beta$ -Adrenomimetik, Isadrin, in an sich
bekannter Weise nachgewiesen. Die $\beta$ -adrenoblockierende
Wirkung des Propranolols, das aus der Diffusionsmatrix eingeführt wurde, kam bereits in 1,5 bis 2 Stunden nach dem Aufkleben der Matrix auf die Haut zum Vorschein, erreichte ihren maximalen Wert in 4 bis 6 Stunden und dauerte innerhalb von sechs Tagen.

An einem Modell von Akonitin-Arrhythmie im Versuch mit weißen rassenlosen Männchen-Ratten wurde die prophylaktische gegenarrhythmische Wirkung der Diffusionsmatrizen mit Propranolol nachgewiesen. In der Kontrolle verursachte die innere Einführung von Akonitin in einer Dosis von 25 $\mu$g/kg in 100% der Fälle in 182$\pm$2,8 Sekunden die Entwicklung einer mono- und polytropen vertikulären Tachyarrhythmie, die im Verlaufe von 51$\pm$3,8 Minuten aufrechterhalten blieb. Es wurden Fälle des Untergangs von Versuchstieren beobachtet. Auf dem Hintergrund der Wirkung von Propranolol bei seiner transdermalen Eindringung aus den Diffusionsmatrizen nach einem Tag nach seiner Applikation verlängerte sich die latente Periode nachweisbar, und die Dauer der Arrhythmie verringerte sich. Die arrhythmische Wirkung des Akonitins zeigte sich lediglich bei einem Teil von Tieren, es wurde kein Untergang von denselben beobachtet. Die schützende gegenarrhythmische Wirkung der Diffusionsmatrizen mit Propranolol bei Arrhythmie, die durch Intoxikation mit Akonitin hervorgerufen wurde, war während 6 Tage nach der Applikation der Matrix an der Haut zu verspüren.

Tabelle 4

| Dauer (h) | Konzentration an Propranolol, ng/ml Tier, Nr. | | | | | |
|---|---|---|---|---|---|---|
| | 1 | 2 | 3 | 4 | 5 | 6 |
| 1 | 2 | 3 | 4 | 5 | 6 | 7 |
| 24–144 | 21,6$\pm$4,9 | 134$\pm$13 | 125$\pm$26 | 179$\pm$35 | 168$\pm$17 | 45,0$\pm$19,3 |
| 24–72 | 24,7$\pm$3,0 | 130$\pm$17 | 117$\pm$34 | 179$\pm$50 | 164$\pm$20 | 57,6$\pm$10,6 |
| 48–96 | 21,6$\pm$2,5 | 127$\pm$13 | 133$\pm$22 | 200$\pm$15 | 162$\pm$19 | 49,6$\pm$18,6 |
| 72–144 | 19,1$\pm$4,2 | 116$\pm$32 | 128$\pm$13 | 185$\pm$21 | 163$\pm$21 | 40,3$\pm$25,4 |

Es wurde von uns eine Diffusionsmatrix "in vitro" untersucht, die auf der Grundlage der erfindungsgemäßen Zusammensetzung hergestellt wurde.

So wird, beispielsweise, auf eine Adhäsionsschicht einer Diffusionsmatrix mit runder Form und einem Radius von 1 cm (Fläche 3,14 cm$^2$), die auf der Grundlage einer Zusammensetzung hergestellt wird, die 26,7 g Propranolol, 40 g Polyäthylenglykol mit einer Molekularmasse von 400 und 100 g Polyvinylpyrrolidon enthält, ein Probestück aus der Epidermis der Leichenhaut eines Menschen (mit der Außenoberfläche der Epidermis an die Diffusionsmatrix) aufgeklebt. Das Laminat der Diffusionsmatrix mit der Hautepidermis taucht man in die in einem Magnetrührer zu vermischende Ringer-Lösung ein. In den vorgegebenen Zeitabständen nimmt man aus der Lösung Proben ab, in denen man den Gehalt an Propranolol mit Hilfe der Spektrofluorometrie ermittelt, wobei die vorher aufgebauten Eichkurven genutzt werden.

Die Prüfergebnisse der Matrizen sind in der Tabelle 5 angeführt.

Die auf der Grundlage der erfindungsgemäßen Zusammensetzung herzustellende Matrix bewirkt eine bedeutende höhere Zufuhrgeschwindigkeit eines Arzneimittels durch die Haut sowie die Freilegung von Propranolol nach der Kinetik der Nullgrößenordnung (mit konstanter Geschwindigkeit). Eine hohe Geschwindigkeit der Freilegung von Propranolol aus der Matrix, die auf der Grundlage der erfindungsgemäßen Zusammensetzung hergestellt wird, ermöglicht es, die Fläche der aufzuklebenden Matrix zu verringern, was ihre Anwendung handlicher macht. Der konstante Geschwindigkeitswert der Zuführung des Arzneistoffes mit der Matrix, die auf der Grundlage der erfindungsgemäßen Zusammensetzung hergestellt wurde, ermöglicht es, die dieselbe während einer längeren Zeit (bis 7 Tagen) auszunutzen, was als Folge den Ausnutzungskoeffizienten des in die Matrix eingeschlossenen Arzneistoffes erhöht.

Die Zusammensetzung der erfindungsgemäßen polymeren Diffusionsmatrix läßt sich in chromatografischen Methoden kontrollieren.

## Tabelle 5

### Untersuchung der Kinetik des Austritts von Propranolol aus der Diffusionsmatrix

| lfd. Nr. | Intervalle der Messung der Konzentration an Propranolol, h | | | | | |
|---|---|---|---|---|---|---|
| | 0 | 0,5 | 1 | 1,5 | 2 | 1,5 |
| 1    2 | 3 | 4 | 5 | 6 | 7 | 8 |
| 1 Menge des Propranolols, das aus der Matrix durch 1 cm$^2$ der Hautoberfläche ausgetreten ist, $\mu$g/cm$^2$ | 0 | 131 | 187 | 133 | 156 | 276 |
| 2 Geschwindigkeit  b/h.cm$^2$ | – | 262 | 112 | 92 | 46 | 40 |

Fortsetzung der Tabelle 5

| lfd. Nr. | Intervalle der Messung der Konzentration an Propranolol, h | | | | | | | |
|---|---|---|---|---|---|---|---|---|
| | 3 | 3,5 | 4 | 6 | 12 | 24 | 48 | 72 |
| 1 | 9 | 10 | 11 | 12 | 13 | 14 | 15 | 16 |
| 1 | 295 | 322 | 345 | 428 | 671 | 1163 | 2123 | 3083 |
| 2 | 38 | 54 | 46 | 41 | 40 | 41 | 40 | 40 |

Zur besseren Erläuterung der beanspruchten Erfindung werden nachstehende Beispiele für ihre konkrete Ausführung angeführt.

Beispiel 1

0,6 g Hydralazin löst man in 330 ml Äthanol, das 33,0 g Polyäthylenglykol mit einer Molekularmasse von 300 enthält, dann setzt man 100 g trockenes Polyvinylpyrrolidon mit einer Molekularmasse von 500 Tsd. zu. Das Gemisch vermischt man bis zur vollständigen Auflösung des Polymeres und der Entstehung einer Lösung mit einem Viskositätsgrad von 400 P.

Die Lösung wird entlüftet und auf eine metallisierte Polyäthylenterephthalat-Unterlage gegossen. Das System trocknet man bei einer Temperatur von 50°C innerhalb von 4 Stunden, wonach man die Oberfläche der hergestellten Matrix mit Antiadhäsions-Schutzpapier laminiert.

Die erhaltene Matrix bewirkt den transdermalen Eintritt von Hydralazin mit einer Geschwindigkeit von 22 $\mu$g/cm$^2$.h. Die Wirkungsdauer beträgt 3 Tage. Der Ausnutzungskoeffizient des Arzneistoffes beträgt 82%. Die Adhäsion mit der Haut beträgt 76 N/m.

Beispiel 2

Man bereitet eine Lösung aus 66 g Propranolol, 100 g Polyäthylenglykol mit einer Molekularmasse von 400 und 100 g Polyvinylpyrrolidon mit einer Molekularmasse von 1000 Tausend in 800 ml Äthanol zu. Man erhält eine Matrix wie in Beispiel 1. Die hergestellte Matrix bewirkt den transdermalen Eintritt von Propranolol mit einer Geschwindigkeit von 37 $\mu$g/cm$^2$.h.

Die Wirkungsdauer beträgt 7 Tage, der Ausnutzungskoeffizient des Arzneistoffes beträgt 65%. Die Adhäsion mit der Haut beträgt 82 n/m.

Beispiel 3

Man bereitet eine Lösung in 400 ml Äthanol aus 25 g Hydralazin, 33,3 g Polyäthylenglykol mit einer Molekularmasse von 600 und 100 g Polyvinylpyrrolidon mit einer Molekularmasse von 1500 Tsd. zu. Man erhält eine Matrix wie in Beispiel 1. Die hergestellte Matrix bewirkt den transdermalen Eintritt von Hydralazin mit einer Geschwindigkeit von 70 $\mu$g/cm$^2$.h. Die Wirkungsdauer beträgt 4 Tage. Der Ausnutzungskoeffizient des Arzneistoffes beträgt 80%. Die Adhäsion mit der Haut beträgt 78 N/cm.

Im Versuch mit Minischweinchen gewährleistet die Matrix in einer Stunde nach dem Aufkleben auf die Haut eine Konzentration an Hydralazin im Blut 50 $\mu$g/ml (Maximaldosis), in der zweiten Stunde verringert sich die Konzentration an Hydralazin im Blut auf 20 $\mu$g/ml und

wird innerhalb von 4 Tagen auf einem konstanten Niveau unterhalten.

Beispiel 4

Man bereitet eine Lösung in 400 ml Äthanol aus 27,2 g Propranolol, 55,2 g Polyäthylenglykol mit einer Molekularmasse von 400 und 100 g Polyvinylpyrrolidon mit einer Molekularmasse von 1500 Tsd. zu. Die erhaltene Matrix gewährleistet den transdermalen Eintritt von Propranolol mit einer Geschwindigkeit von 47 $\mu$g/cm$^2$.h. Die Wirkungsdauer beträgt 6 Tage, der Ausnutzungskoeffizient des Arzneistoffes beträgt 80%. Die Adhäsion mit der Haut beträgt 76 N/m.

Beispiel 5

In 400 ml 4%iger Lösung des Nitroglyzerins im Äthanol löst man 50 g Polyäthylenglykol mit einer Molekularmasse von 400 und 100 g Polyvinylpyrrolidon mit einer Molekularmasse von 500 Tds. auf. Man erhält eine Matrix wie in Beispiel 1. Die hergestellte Matrix gewährleistet den transdermalen Eintritt von Nitroglyzerin mit einer Geschwindigkeit von 39 $\mu$g/cm$^2$.h. Die Wirkungsdauer beträgt 4 Tage, der Ausnutzungskoeffizient des Arzneistoffes beträgt 79%. Die Adhäsion mit der Haut beträgt 73 N/m.

Im Versuch mit Kaninchen bewirkt die Matrix die Unterhaltung einer unverändert bleibenden Konzentration an Nitroglyzerin im Blut innerhalb von 4 Tagen auf einem Durchschnittsniveau von 2,5 ng/ml.

Beispiel 6

Man bereitet eine Lösung in 350 ml Äthanol aus 17 g Nitrosorbid, 50 g Polyäthylenglykol mit einer Molekularmasse von 400 und 100 g Polyvinylpyrrolidon mit einer Molekularmasse von 1500 Tsd. zu. Man erhält eine Matrix wie in Beispiel 1. Die hergestellte Matrix gewährleistet den transdermalen Eintritt des Nitrosorbids mit einer Geschwindigkeit von 35 $\mu$g/cm$^2$.h. Die Wirkungsdauer beträgt 5 Tage, der Ausnutzungskoeffizient des Arzneimittels beträgt 75%. Die Adhäsion mit der Haut beträgt 70 g/cm.

Die erhaltene Matrix gewährleistet die Unterhaltung der Konzentration an Nitrosorbid im Blutplasma von Ka-

ninchen innerhalb von 5 Tagen auf einem konstanten Durchschnittsniveau von 27,5 ng/ml.

Beispiel 7

Man bereitet eine Lösung in 500 ml Äthanol aus 26,7 g Propranolol, 40 g Polyäthylenglykol mit einer Molekularmasse von 400 und 100 g Polyvinylpyrrolidon mit einer Molekularmasse von 1000 Tausend zu. Man erhält eine Matrix wie in Beispiel 1. Die erhaltene Matrix bewirkt den transdermalen Eintritt von Propranolol mit einer Geschwindigkeit von 25 $\mu$g/cm$^2$.h. Die Wirkungsdauer beträgt 5 Tage, der Ausnutzungskoeffizient des Arzneistoffes beträgt 62%. Die Adhäsion mit der Haut beträgt 47 N/m.

Beispiel 8

Man bereitet eine Lösung in 400 ml Äthanol aus 25 g Propranolol, 3,6 g 1-Dodezyl-(azazykloheptanon-2), 50 g Polyäthylenglykol mit einer Molekularmasse von 600 und 100 g Polyvinylpyrrolidon mit einer Molekularmasse von 1500 Tausend zu. Man erhält eine Matrix wie in Beispiel 1. Die erhaltene Matrix bewirkt den transdermalen Eintritt von Propranolol mit einer Geschwindigkeit von 51 $\mu$g/cm$^2$.h. Die Wirkungsdauer beträgt 3 Tage, der Ausnutzungskoeffizient des Arzneistoffes beträgt 72%. Die Adhäsion mit der Haut beträgt 76 N/m.

Beispiel 9

Man bereitet eine Lösung in 450 ml Äthanol aus 11,7 g Nitrosorbid, 5 g N,N-Dimethyl-m-toluamid, 50 g Polyäthylenglykol mit einer Molekularmasse von 400 und 100 g Polyvinylpyrrolidon mit einer Molekularmasse von 1200 Tausend zu. Man erhält eine Matrix wie in Beispiel 1. Die erhaltene Matrix bewirkt den transdermalen Eintritt von Nitrosorbid mit einer Geschwindigkeit von 40 $\mu$g/cm$^2$.h. Die Wirkungsdauer beträgt 3 Tage, der Ausnutzungskoeffizient des Arzneistoffes beträgt 69%. Die Adhäsion mit der Haut beträgt 70 N/m.

Beispiel 10

In 160 g 4%iger Lösung des Nitroglyzerins in Äthanol löst man 4,8 g Dimethylsulfoxid, 47,6 g Polyäthylenglykol mit einer Molekularmasse von 400 und 100 g Polyvinylpyrrolidon mit einer Molekularmasse von 1500 Tsd. auf. Man erhält eine Matrix wie in Beispiel 1. Die erhaltene Matrix bewirkt den transdermalen Eintritt von Nitroglyzerin mit einer Geschwindigkeit von 42 $\mu$g/cm$^2$.h. Die Wirkungsdauer beträgt 3 Tage, der Ausnutzungskoeffizient des Arzneistoffes beträgt 77%. Die Adhäsion mit der Haut beträgt 5 N/m.

Beispiel 11

Man bereitet eine Lösung in 400 ml Äthanol aus 25 g Propranolol, 3,6 g N-Methylpyrrolidon, 50 g Polyäthylenglykol mit einer Molekularmasse von 600 und 100 g Polyvinylpyrrolidon mit einer Molekularmasse von 1600 Tsd. zu. Man erhält eine Matrix wie in Beispiel 1. Die erhaltene Matrix bewirkt den transdermalen Eintritt von Propranolol mit einer Geschwindigkeit von 50 $\mu$g/cm$^2$.h. Die Wirkungsdauer beträgt 6 Tage, der Ausnutzungskoeffizient des Arzneimittels beträgt 70%. Die Adhäsion mit der Haut beträgt 80 N/m.

Beispiel 12

Man bereitet eine Lösung in 350 ml Äthanol aus 16,7 g Nitrosorbid, 10 g Tween-80, 40 g Polyäthylenglykol mit einer Molekularmasse von 500 und 100 g Polyvinylpyrrolidon mit einer Molekularmasse von 1500 Tausend zu. Man erhält eine Matrix wie in Beispiel 1. Die erhaltene Matrix bewirkt den transdermalen Eintritt von Nitrosorbid mit einer Geschwindigkeit von 37 $\mu$g/cm$^2$.h. Die Wirkungsdauer beträgt 5 Tage, der Ausnutzungskoeffizient des Arzneistoffes beträgt 71%. Die Adhäsion mit der Haut beträgt 67 N/m.

Beispiel 13

Man bereitet eine Lösung in 400 ml Äthanol aus 66 g Propranolol, 33 g Polyäthylenglykol mit einer Molekularmasse von 400 und 100 g Polyvinylpyrrolidon mit einer Molekularmasse von 1500 Tsd. zu. Das Gemisch wird bis zur vollständigen Auflösung vermischt und die Lösung entlüftet. Auf eine Polyäthylenterephthalat-Folie werden 5 g Papier mit einem Verhältnis der Wasseraufnahme zur Masse gleich 6,06 gelegt und auf diese wird die vorher zubereitete polymere Lösung aufgetragen. Das System trocknet

- 22 -

man bei einer Temperatur von 50°C innerhalb von 4 Stunden, wonach man die Oberfläche der hergestellten Matrix mit silikonisiertem Antiadhäsion-Schutzpapier laminiert. Die erhaltene Matrix bewirkt den transdermalen Eintritt von Propranolol mit einer Geschwindigkeit von 48 $\mu$g/cm$^2$.h. Die Wirkungsdauer beträgt 7 Tage, der Ausnutzungskoeffizient des Arzneistoffes beträgt 71%. Die Adhäsion mit der Haut beträgt 62 N/m.

Beispiel 14

Man bereitet eine Lösung in 500 ml Äthanol aus 0,6 g Propranolol, 100 g Polyäthylenglykol mit einer Molekularmasse von 400 und 100 g Polyvinylpyrrolidon mit einer Molekularmasse von 500 Tsd. zu. Als Füllstoff verwendet man 200 g eines gewebten Baumwollstoffes mit einem Verhältnis der Wasseraufnahme zur Masse gleich 2,18. Man erhält eine Matrix wie in Beispiel 13. Die erhaltene Matrix bewirkt den transdermalen Eintritt von Propranolol mit einer Geschwindigkeit von 49 $\mu$g/cm$^2$.h. Die Wirkungsdauer beträgt 2 Tage, der Ausnutzungskoeffizient des Arzneistoffes beträgt 81%. Die Adhäsion mit der Haut beträgt 82 N/m.

Beispiel 15

Man bereitet eine Lösung in 500 ml Äthanol aus 28,6 g Propranolol, 63,8 g Polyäthylenglykol mit einer Molekularmasse von 400 und 100 g Polyvinylpyrrolidon mit einer Molekularmasse von 1500 Tsd. zu. Als Füllstoff verwendet man 170 g eines Baumollstoffes mit einem Verhältnis der Wasseraufnahme zur Masse gleich 2,57. Man erhält eine Matrix wie in Beispiel 13. Die erhaltene Matrix bewirkt den transdermalen Eintritt von Propranolol mit einer Geschwindigkeit von 51 $\mu$g/cm$^2$.h. Die Wirkungsdauer beträgt 6 Tage, der Ausnutzungskoeffizient des Arzneistoffes beträgt 78%. Die Adhäsion mit der Haut beträgt 78 N/m.

Beispiel 16

Man erhält eine Matrix gemäß Beispiel 15, wobei man als Füllstoff 68 g eines Bauwollstoffes mit einem Verhältnis der Wasseraufnahme zur Masse gleich 2,33 verwendet. Die erhaltene Matrix bewirkt den transdermalen Eintritt von Propranolol mit einer Geschwindigkeit von 49 $\mu$g/cm$^2$.h. Die Wirkungsdauer beträgt 5 Tage, der Ausnutzungskoeffizient des Arzneistoffes beträgt 77%. Die Adhäsion mit der Haut beträgt 75 N/m.

Beispiel 17

Man erhält eine Matrix gemäß Beispiel 15, wobei man als Füllstoff 39 g eines Baumwollstoffes mit einem Verhältnis der Wasseraufnahme zur Masse gleich 6,98 verwendet. Die erhaltene Matrix bewirkt den transdermalen Eintritt von Propranolol mit einer Geschwindigkeit von 55 $\mu$g/cm$^2$.h. Die Wirkungsdauer beträgt 7 Tage, der Ausnutzungskoeffizient des Arzneistoffes beträgt 79%. Die Adhäsion mit der Haut beträgt 77 n/m.

Beispiel 18

Man erhält eine Matrix gemäß Beispiel 15, wobei man als Arzneistoff 28,6 g Verapamyl und als Füllstoff 51 g eines Baumwollstoffes mit einem Verhältnis der Wasseraufnahme zur Masse gleich 6,98 verwendet. Die erhaltene Matrix bewirkt den transdermalen Eintritt von Verapamyl mit einer Geschwindigkeit von 19 $\mu$g/cm$^2$.h. (ohne Füllstoff - $\mu$g/cm$^2$.h). Die Wirkungsdauer beträgt 6 Tage, der Ausnutzungskoeffizient des Arzneistoffes beträgt 74%. Die Adhäsion mit der Haut beträgt 80 n/m.

Beispiel 19

Man erhält eine Matrix gemäß Beispiel 15, wobei man als Arzneistoff 28,6 g Nifedipin und als Füllstoff 37 g eines Baumwollstoffs mit einem Verhältnis der Wasseraufnahme zur Masse gleich 6,98 verwendet. Die erhaltene Matrix bewirkt die transdermale Zuführung von Nifedipin mit einer Geschwindigkeit von 6,5 $\mu$g/cm$^2$.h (ohne Füllstoff - 4,2 $\mu$g/cm$^2$.h). Die Wirkungsdauer beträgt 6 Tage, der Ausnutzungskoeffizient des Arzneistoffes beträgt 72%. Die Adhäsion mit der Haut beträgt 77 n/m.

Industrielle Anwendbarkeit

Die angemeldete Erfindung kann in der pharmazeutischen Industrie und in der Landwirtschaft als Arzneiform prolongierter Wirkung mit kontrollierbarer Zufuhrgeschwindigkeit eines Arzneistoffes dem Organismus eines Menschen oder Tieres eingesetzt werden.

0340307

PATENTANSPRÜCHE

1. Zusammensetzung einer polymeren Diffusionsmatrix für transdermale Einführung von Arzneistoffen, die ein Bindemittel, Polyvinylpyrrolidon, ein Plastifizierungsmittel und einen Arzneistoff enthält, d a d u r c h  g e k e n n z e i c h n e t, daß die Zusammensetzung als Bindemittel Polyvinylpyrrolidon mit einer Molekularmasse von 500 Tsd. bis 1500 Tsd. und als Plastifizierungsmittel Polyäthylenglykol mit einer Molekularmasse von 300 bis 600 bei folgendem Verhältnis der genannten Komponenten in Masseanteilen enthält:

| | |
|---|---|
| Polyvinylpyrrolidon | 100 |
| Polyäthylenglykol | von 33 bis 100 |
| Arzneistoff | von 0,6 bis 66 |

2. Zusammensetzung nach Anspruch 1, d a d u r c h  g e k e n n z e i c h n e t, daß sie zusätzlich einen hydrophilen unlöslichen faserigen Füllstoff auf der Grundlage von Zellulose in einer Menge von 5 bis 500 Masseanteilen enthält.

3. Zusammensetzung nach jedem der Ansprüche von 1 bis 2, d a d u r c h  g e k e n n z e i c h n e t, daß sie zusätzlich Permeabilitätspromotoren der Arzneistoffe durch die Haut in einer Menge von 1 bis 10 Masseanteilen enthält.

4. Zusammensetzung nach jedem der Ansprüche von 1 bis 3, d a d u r c h  g e k e n n z e i c h n e t, daß sie zusätzlich Lösungsmittel der Arzneistoffe in einer Menge von 5 bis 15 Masseanteilen enthält.

# INTERNATIONAL SEARCH REPORT

International Application No PCT/SU 88/00104

| I. CLASSIFICATION OF SUBJECT MATTER (if several classification symbols apply, indicate all) |
|---|

According to International Patent Classification (IPC) or to both National Classification and IPC

IPC⁴ C 08 L 39/06, C 08 K 7/02, A 61 K 9/70, A 61 L 15/03, A 61 M 37/00

## II. FIELDS SEARCHED

| Minimum Documentation Searched [7] | |
|---|---|
| Classification System | Classification Symbols |
| IPC⁴ | C 08 L 39/06, C 08 K 7/02, A 61 K 9/00, 9/70, A 61 L 15/03, 15/06, A 61 M 37/00 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | US, A, 4460562, (Key Pharmaceuticals, Inc.), 17 July 1984 (17.07.84), see the claims | 1 |
| A | US, A, 4605548, (Nitto Electric Industrial Co., Ltd), 12 August 1986 (12.08.86), see columns 3,4 | 1-2 |
| A | GB, A, 2095108, (Nitto Electric Industrial Co. Ltd.), 29 September 1982 (29.09.82), see page 3, lines 10-39, the abstract | 1-4 |
| A | WO, A1, 82/00099, (KEY PHARMACEUTICALS, INCORPORATED), 21 January 1982 (21.01.82), see page 4, the abstract | 1-4 |
| A | WO, A1, 86/02272, (KEY PHARMACEUTICALS, INC,), 24 April 1986 (24.04.86), see pages 17,18, the abstract | 1-2 |

--------------------

\* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 18 October 1988 (18.10.88) | 24 November 1988 (24.11.88) |
| International Searching Authority | Signature of Authorized Officer |
| ISA/SU | |

Form PCT/ISA/210 (second sheet) (January 1985)